# EUROPÄISCHE PATENTSCHRIFT

(11) **EP 3 760 168 B1**
(45) Veröffentlichungstag und Bekanntmachung des Hinweises auf die Patenterteilung: **27.07.2022**
(21) Anmeldenummer: 20020279.4
(22) Anmeldetag: 16.06.2020
(51) Int. Cl.: A61F 5/01

(54) **ORTHESE MIT EINER EIN BIKONDYLÄRES GELENK NACHBILDENDEN GELENKEINRICHTUNG UND GELENKEINRICHTUNG**
ORTHOSIS WITH AN ARTICULATED DEVICE MIMICKING A BICONDYLAR JOINT AND ARTICULATED DEVICE
ORTHÈSE DOTÉE D'UN DISPOSITIF ARTICULAIRE REPRODUISANT UNE ARTICULATION BICONDYLIENNE ET DISPOSITIF ARTICULAIRE

(30) Priorität: 17.06.2019 DE 102019116417
(43) Veröffentlichungstag der Anmeldung: 06.01.2021
(73) Patentinhaber: Wagner, Michael, 91522 Ansbach (DE); Kobolla, Harald, 90556 Seukendorf (DE)
(72) Erfinder: Wagner, Michael, 91522 Ansbach (DE); Kobolla, Harald, 90556 Seukendorf (DE)

(56) Entgegenhaltungen:
- WO-A1-2019/109178
- US-A- 5 542 774
- US-A1- 2002 026 136
- US-A1- 2009 030 356

## Beschreibung

Die Erfindung beschreibt Gelenkeinrichtung zur Stabilisierung eines bikondylären Gelenks des menschlichen oder tierischen Körpers sowie eine Knieorthese mit zwei derartigen Gelenkeinrichtungen.

Aus der DE 196 05 734 A1 ist eine Kniegelenkorthese bekannt mit einem Unterschenkelteil, einem Oberschenkelteil sowie einem lateralen und einem medialen Orthesengelenk, die das Unterschenkelteil und das Oberschenkelteil miteinander verbinden, wobei das laterale Orthesengelenk von einem Gelenk gebildet ist, das besteht aus zwei ineinandergreifenden Zahnrädern, deren Achsen beidseitig mit jeweils einer Gelenkplatte fest verbunden sind, wobei jedes der beiden Zahnräder jeweils eine Strebe aufweist, die zur festen Verbindung des lateralen Orthesengelenks mit dem Unterschenkelteil und dem Oberschenkelteil dient, das mediale Orthesengelenk von einem einachsigen Gelenk gebildet ist, das besteht aus einem U-förmigen Oberteil, das ein Unterteil umfaßt, wobei Oberteil und Unterteil gelenkig durch einen Zapfen miteinander verbunden sind.

Die DE199 45 829 A1 offenbart ein Knieorthesengelenk mit Befestigungsmitteln für das Ober- und Unterschenkelteil einer Orthese, die über mindestens eine zweiteilige Schiene den Ober- und Unterschenkel verbindet, wobei ein Ober- und Unterschenkelteil aufeinander abrollen, wobei die gelenkbildenden Bahnkurven eines Oberschenkelteiles und Unterschenkelteiles zur Gewährleistung des notwendigen Formschlusses über einen Steg mit Führungen verbunden sind. Der Steg ist mittels eines Lagerzapfens am Oberschenkelteil gelagert und durch die Führungszapfen und die Führungen am Oberschenkelteil oder Unterschenkelteil oder Steg mit einem Ober- und Unterschenkelteil verbunden, so daß eine kinematisch zwangsgeführte Bewegung eines Ober- und Unterschenkelteiles erfolgt.

Die DE 10 2004 046 743 A1 offenbart eine Knieorthese mit einem femuralen und einem tibialen Orthesenteil, die über seitliche Gelenke miteinander verbunden sind, wobei die Abwinklung der Gelenke jeweils durch zwei Führungsbahnen, insbesondere Führungsschlitze, einerseits und diesen jeweils zugeordnete Nocken, insbesondere Stiftfolger, andererseits definiert ist, wobei die Führungsbahnen, insbesondere Führungsschlitze, sich längs zweier sich in einem Punkt schneidender Rastpolkurven, insbesondere Rastpolkreise, erstrecken.

Die US 5 542 774 A offenbart ein Orthesengelenk, umfassend ein erstes Element, ein zweites Element und ein Kunststoffzwischenelement, die durch einen Drehpunkt miteinander verbunden sind. Das zweite Element umfasst einen daran befestigten Nockenstößel. Das erste Element und das mittlere Kunststoffelement umfassen jeweils einen ersten und einen zweiten bogenförmigen Schlitz. Der erste und der zweite bogenförmige Schlitz sind zueinander ausgerichtet. Das erste und das zweite Element sind gegeneinander drehbar. Der Nockenstößel ragt durch den zweiten Schlitz in den ersten Schlitz hinein. Der Bewegungsbereich wird durch die Länge des Schlitzes relativ zur Größe des Nockenfolgers bestimmt.

In Kenntnis des Standes der Technik liegt der Erfindung die Aufgabe zugrunde, eine Gelenkeinrichtung für eine Orthese und eine Orthese hiermit vorzustellen, wobei das Gelenk die bikondylären Bewegung in allen Raumrichtungen des Kniegelenks nachbildet.

Diese Aufgabe wird erfindungsgemäß gelöst durch eine Gelenkeinrichtung zur Stabilisierung eines bikondylären Gelenks des menschlichen oder tierischen Körpers mit einem ersten und einem zweiten Gelenkarm, mit einem Elastomerformkörper und mit einer Verbindungseinrichtung, wobei der erste Gelenkarm einen ersten Endabschnitten mit einer erste Hauptfläche, die eine ersten Normalenrichtung aufweist, und die einen ersten Endabschnittausnehmung aufweist, wobei der zweite Gelenkarm einen zweiten Endabschnitt mit einer zweiten Hauptfläche, die eine zweiten Normalenrichtung aufweist, die mit der ersten Normalenrichtung einen Zwischenwinkel einschließt, der vorzugsweise kleiner 10° beträgt, und die eine zweite Endabschnittausnehmung aufweist, und wobei der der erste und zweite Gelenkarm im Bereich ihrer jeweiligen Endabschnitte überlappend angeordnet sind, wobei der Elastomerformkörper einen ersten Teilkörper aufweist, der im Bereich der Endabschnitte der Gelenkarme zwischen diesen angeordnet ist und eine Elastomerformkörperausnehmung aufweist, die mit der ersten Endabschnittausnehmung zumindest teilweise in erster Normalenrichtung fluchtet, wobei die Verbindungseinrichtung den ersten Gelenkarm zum zweiten Gelenkarm derart beweglich fixiert, dass die Gelenkarme gegeneinander um die erste Normalenrichtung als Drehachse der Bewegung des Gelenks folgend verdrehbar sind und hierzu einer der Gelenkarme in seinem Endabschnitt einen Zapfen aufweist, der in eine Nut des Elastomerformkörpers eingreift und während der Bewegung dieser Nut folgend die Form der Bewegung bestimmt und dass hierbei die beiden Gelenkarme eine Kippbewegung zueinander ausführen können, wobei sich der Wert des Zwischenwinkels verändert.

Wesentlich bei der Drehbewegung mit der ersten Normalenrichtung als Drehachse ist, dass die Drehachse nicht starr zu einem der beiden Gelenkarme ausgerichtet ist, sondern dass durch die Führung des Zapfens in der Nut die Drehachse während der Drehbewegung ihre Lage zu den Gelenkarmen senkrecht zur Normalenrichtung veränderbar ist.

Es ist bevorzugt, wenn die Verbindungseinrichtung einen Abschnitt aufweist, der durch die erste Endabschnittausnehmung und die Elastomerformkörperausnehmung hindurchreicht. Hierbei kann die Elastomerformkörperausnehmung senkrecht zur ersten Normalenrichtung beweglich in dem Elastomerformkörper angeordnet sein. Hierbei ist es besonders bevorzugt, wenn die erste Endabschnittausnehmung eine Ausdehnung senkrecht zur ersten Normalenrichtung aufweist, die größer ist als der darin angeordnete Abschnitt der Verbindungseinrichtung.

Vorteilhafterweise ist der der Elastomerformkörper ausgebildet aus einem Material der Materialgruppe der Elastomere, bevorzugt der Silikonkautschuke, insbesondere aus thermisch stabilisiertem Silikonkautschuk, insbesondere mit einer Shore-A-Härte zwischen 55 und 90, bevorzugst zwischen 70 und 80 oder aus einem Material der Materialgruppe der modifizierten PTFEs, bevorzugt der gefülltem PTFE, insbesondere aus mit Silikat gefülltem PTFE, insbesondere mit einem Kaltstauchwert zwischen 3% und 40%, insbesondere zwischen 3% und 20%

Es kann auch vorteilhaft sein, wenn ein zweiter Teilkörper des Elastomerformkörpers in eine Ausnehmung eines Endabschnitts eines Gelenkarms, vorzugsweise verdrehsicher, angeordnet ist.

Es kann ebenfalls vorteilhaft sein, wenn ein dritter Gelenkarm oder Teilgelenkarm mit einem dritten Endabschnitt an dem ersten Gelenkarm fixiert angeordnet ist, wobei zweiten Endabschnitt und der Elastomerformkörper zwischen dem ersten und dritten Endabschnitt angeordnet sind. Eine Gelenkeinrichtung mit einem derartigen dritten Gelenkarm oder Teilgelenkarm soll im Folgenden als zweite erfindungsgemäße Art der Gelenkeinrichtung bezeichnet werden, während eine Gelenkeinrichtung ohne diesen dritten Gelenkarm oder Teilgelenkarm als erste Art bezeichnet wird.

Insbesondere kann die Verbindungseinrichtung als Schraubverbindung oder als Nietverbindung ausgebildet sein.

Die Erfindung beschreibt weiterhin eine Orthese mit einer ein bikondyläres Gelenk nachbildenden oben beschriebenen Gelenkeinrichtung mit einer ersten und einer zweiten Manschette, die jeweils eine Fixiereinrichtung zur Fixierung der Orthese an einer Extremität mit einem bikondylären Gelenk, das eine Gelenkdrehachse aufweist, des menschlichen oder tierischen Körpers aufweisen, wobei die erste Manschette mittels der Gelenkeinrichtung mit der zweiten Manschette verbunden ist und die erste und zweite Normalenrichtung im Wesentlichen parallel zur Gelenkdrehachse ausgerichtet sind.

Besonders vorteilhaft an dieser erfindungsgemäßen Orthese ist, dass sie während der Bewegung der Gelenkeinrichtung eine Verschiebung der Drehachse in einer Richtung senkrecht zu ihrer Orientierung mit einer Kippung der Drehachse kombiniert, ohne dass die beiden Bewegungen gekoppelt zueinander sind. In anderen Worten, die Verschiebung der Drehachse ist unabhängig von ihrer Verkippung. Beide Bewegungen sind in der Regel durch die Antonie und damit durch die natürliche Bewegung des bewegenden Gelenks vorgegeben und nicht zwangsweise durch die Gelenkeinrichtung gekoppelt.

Hierbei kann es bevorzugt sein, wenn gegenüberliegend jeweils eine Gelenkeinrichtung angeordnet ist.

Es kann besonders vorteilhaft sein, wenn die Orthese als Knieorthese, die erste Manschette als Oberschenkelmanschette und die zweite Manschette als Unterschenkelmanschette ausgebildet sind.

Alle hier im Singular genannten Merkmale können selbstverständlich, soweit technisch möglich und vorteilhaft, auch mehrfach vorhanden sein. Es versteht sich weiterhin, dass die verschiedenen Ausgestaltungen der Erfindung, gleich ob sie im Rahmen der Gelenkeinrichtung oder der Orthese genannt sind, einzeln oder in beliebigen Kombinationen realisiert sein können, um Verbesserungen zu erreichen. Insbesondere sind die vorstehend genannten und erläuterten, sich nicht selbstverständlich ausschließenden, Merkmale, insbesondere betreffend die beiden Arten der Gelenkeinrichtung, nicht nur in den angegebenen Kombinationen, sondern auch in anderen Kombinationen oder in Alleinstellung einsetzbar, ohne den Rahmen der vorliegenden Erfindung zu verlassen.

Weitere Erläuterungen der Erfindung, vorteilhafte Einzelheiten und Merkmale ergeben sich aus der nachfolgenden Beschreibung der in den Fig. 1 bis 8 dargestellten Ausführungsbeispiele der Erfindung oder von Teilen hiervon. Dabei zeigt:
Figur 1 zeigt eine Gelenkeinrichtung der ersten erfindungsgemäßen Art.
Figur 2 und 3 zeigen eine Gelenkeinrichtung der zweiten erfindungsgemäßen Art.
Figur 4 zeigt einen Elastomerformkörper einer Gelenkeinrichtung der ersten erfindungsgemäßen Art.
Figuren 5 bis 8 zeigen verschiedene Ansichten einer Gelenkeinrichtung der zweiten erfindungsgemäßen Art.

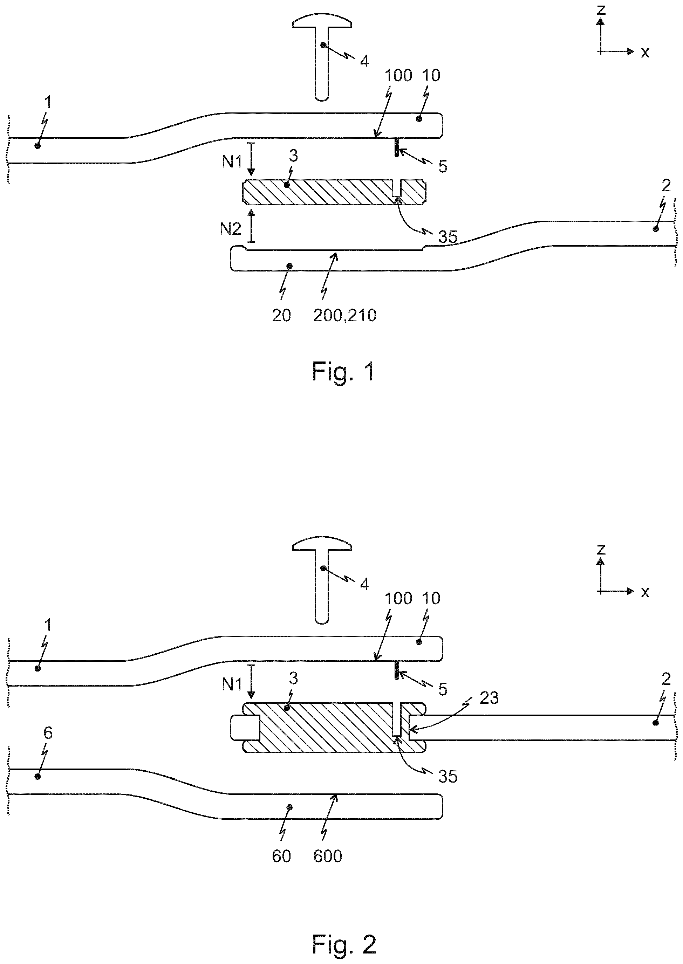

Figur 1 zeigt in Explosionsdarstellung eine Gelenkeinrichtung der ersten erfindungsgemäßen Art. Dargestellt ist ein Abschnitt eines erster Gelenkarms 1, der als hier als metallischer, flächiger Formkörper ausgebildet ist. Der erste Gelenkarm 1 weist einen ersten Endabschnitt auf 10, dessen erste Hauptfläche 100 eine ersten Normalenrichtung N1, hier in negative z-Richtung, aufweist.

Dargestellt ist weiterhin ein Abschnitt eines zweiten Gelenkarms 2, der ebenfalls als metallischer, flächiger Formkörper ausgebildet ist. Alternativ kann der Werkstoff dieses, wie auch des ersten oder weiterer Gelenkarme ein Kunststoff, insbesondere ein Kohlefaserverbundwerkstoff, sein. Der zweite Gelenkarm 2 weist einen zweiten Endabschnitt 20 auf, dessen zweite Hauptfläche 200 eine zweite Normalenrichtung N2, hier in positive z-Richtung, aufweist. Somit liegen sich die beiden Hauptflächen 100,200 gegenüber und weisen erfindungsgemäß einen Abstand in z-Richtung voneinander auf.

Der Bereich zwischen den beanstandeten Hauptflächen 100,200 der beiden Gelenkarme 1,2, genauer deren Endabschnitt 10,20 ist erfindungsgemäß dafür vorgesehen einen Elastomerformkörper 3 oder zumindest einen Teilkörper dieses Elastomerformkörpers anzuordnen. Der Elastomerformkörper 3 ist hier ausgebildet als aus einem mit Silikat gefülltem PTFE mit einem Kaltstauchwert von 10%. Er kann zudem auch hybrid ausgebildet sein mit verschiedenen Bereichen, wobei ein erster zentraler Bereich einem Kaltstauchwert von 5% und ein zweiter, umgebender Bereich einen Kaltstauchwert von 10% aufweist.

Der erste Endabschnitt 10, der Elastomerformkörper 3 und der zweite Endabschnitt 20 bilden hier gewissermaßen einen Stapel aus. Es kann auch vorteilhaft sein, wenn mindestens eine der Hauptflächen 100,200, dargestellt ist dies an der zweiten Hauptfläche 200 eine Vertiefung 210 aufweist, in der ein zweiter Teilkörper, der hier ohne Beschränkung der Allgemeinheit, als ein flächiger Abschnitt des Elastomerformkörpers 3 ausgebildet ist, angeordnet sein. Dies dient insbesondere der verdrehsicheren Anordnung des Elastomerformkörpers 3.

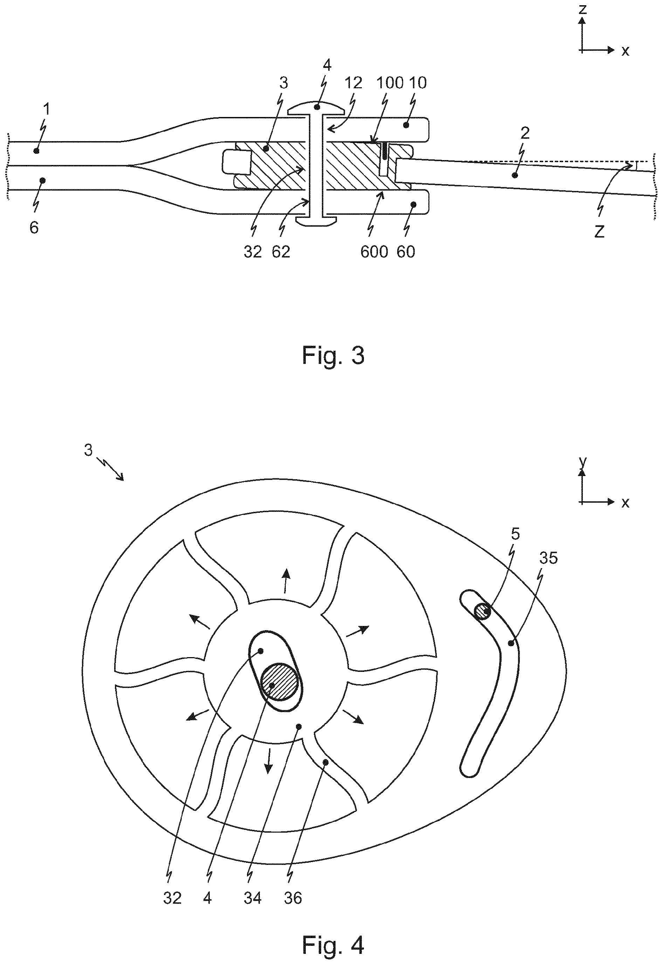

Weiterhin dargestellt ist eine Verbindungseinrichtung 4, hier ausgebildet als Nietverbindung, die den erste Endabschnitt 10, den Elastomerformkörper 3 und den zweiten Endabschnitt 20 miteinander verbindet und zueinander fixiert. Die Verbindungseinrichtung 3, also die Niete der Nietverbindung, reicht, vgl. auch Figur 3, durch die erste Endabschnittausnehmung 12, eine Elastomerformkörperausnehmung 32 und durch eine zweite Endabschnittausnehmung.

Hierdurch fixiert die Verbindungseinrichtung 4 den ersten Gelenkarm 1 zum zweiten Gelenkarm 2 derart beweglich, dass einerseits die Gelenkarme 1,2 gegeneinander um die erste Normalenrichtung N1 als Drehachse der Bewegung des Gelenks folgend verdrehbar sind und, dass andererseits die beiden Gelenkarme 1,2 eine Kippbewegung zueinander ausführen können, wobei sich der Wert des Zwischenwinkels Z, vgl. Figur 3, zwischen den beiden Normalenrichtungen N1 ,N2 verändert. Die Verbindungseinrichtung 4 ist hierzu im ersten Endabschnitt 10 senkrecht zur ersten Normalenrichtung N1 in ein oder beide Raumrichtungen beweglich angeordnet. Hierzu weist der erste Endabschnitt 10 eine Endabschnittausnehmung 12 auf, durch die eine Abschnitt der Verbindungseinrichtung 4 hindurchreicht, der in der jeweiligen Raumrichtung großer ausgebildet ist als der zugeordnete Abschnitt der Verbindungseinrichtung 4. Für eine Raumrichtung bedeutet diese bei einer runden Ausgestaltung des Abschnitts der Verbindungseinrichtung 4, dass die zugeordnete erste Endabschnittausnehmung 12 als Langloch ausgebildet ist. Somit können die beiden Gelenkarme 1,2 eine Kippbewegung zueinander ausführen, wobei sich der Wert eines Zwischenwinkels Z, zwischen den beiden Normalenrichtungen im Verlauf der Bewegung verändern kann.

Zur Ausführung der erstgenannten Bewegung weist einer der Gelenkarme 1,2, hier der erste, in seinem Endabschnitt 10 einen Zapfen 5 auf, der in eine Nut 35 des Elastomerformkörpers 3, die auch als Vertiefung oder auch durchgehend durch den gesamten Elastomerformkörper ausgebildet sein kann, eingreift und während der Bewegung der Zapfen 5 dieser Nut 35 folgend die Form der Bewegung bestimmt.

Figur 2 und 3 zeigen eine Gelenkeinrichtung der zweiten erfindungsgemäßen Art, wobei die Funktionalität grundsätzlich die gleiche ist wie bei der ersten oben unter Figur 1 beschriebenen Art. Figur 2 zeigt in Explosionsdarstellung einen ersten Gelenkarm 1, grundsätzlich gleich dem gemäß Figur 1. Weiterhin dargestellt ist ein dritter Gelenkarm 6 mit einem dritten Endabschnitt 60 und einer, der ersten zugewandte, dritten Hauptfläche 600. Beide Gelenkarme 1,6 liegen aufeinander auf und sind zueinander fixiert. Sie weisen jeweils eine Kröpfung auf, die im Bereich der jeweiligen Endabschnitte 10,60 einen Abstand zwischen der ersten und dritten Hauptfläche 100,600 ausbildt.

In diesem gabelartigen Zwischenraum zwischen der ersten und dritten Hauptfläche 100,600 ist ein zweiter Gelenkarm 2 mit seinem zweiten Endabschnitt 20 angeordnet. Dieser zweiten Endabschnitt 20 weist eine Ausnehmung 23, genauer eine Elastomerformkörperausnehmung, auf, in der ein zweiter Teilkörper des Elastomerformkörpers 3 verdrehsicher angeordnet ist. Ein erster Teilkörper des Elastomerformkörpers 3 ist zwischen dem zweiten Endabschnitt 20 und dem ersten Endabschnitt 10, und ein weiterer erster Teilkörper des Elastomerformkörpers 3 ist zwischen dem zweiten Endabschnitt 10 und dem dritten Endabschnitt 60 angeordnet.

Die Verbindungseinrichtung 4, hier wiederum eine Nietverbindung, verbindet den ersten Endabschnitt 10 mit dem dritten Endabschnitt 60 und ist gegenüber diesen nicht beweglich angeordnet. Vielmehr weist hier der Elastomerformkörper 3 eine Elastomerformkörperausnehmung 32 auf, durch die ein Abschnitt der Verbindungseinrichtung 4 hindurchreicht und die innerhalb des Elastomerformkörpers 3 senkrecht zur ersten Normalenrichtung beweglich angeordnet ist, vgl. auch Figur 4.

Zur Ausführung der erstgenannten Bewegung weist einer der Gelenkarme 1, hier der erste, in seinem Endabschnitt 10 einen Zapfen 5 auf, der in eine Nut 35 des Elastomerformkörpers 3, die als Vertiefung oder auch durchgehend durch den gesamten Elastomerformkörper 3 ausgebildet sein kann, eingreift und während der Bewegung der Zapfen 5 dieser Nut 35 folgend die Form der Bewegung bestimmt.

Figur 3 zeigt den zweiten Gelenkarm 2 um den Zwischenwinkel Z verkippt gegenüber dem ersten und hier auch dem dritten Gelenkarm 1,6. Eine derartige Verkippung ist zur Anpassung der Gelenkeinrichtung an die Anatomie des Trägers einer damit ausgestatteten Orthese sinnvoll und in manchen Anwendungsfällen notwendig um das jeweilige Gelenk in seiner natürlichen Bewegung nicht zu behindern.

Figur 4 zeigt in Draufsicht einen flächigen Elastomerformkörper 3 einer Gelenkeinrichtung. Dieser weist eine nichtrunde Grundform auf, um bei der Anordnung in oder zu einem Gelenkarm dort verdrehsicher angeordnet werden zu können.

Weiterhin weist der Elastomerformkörper 3 eine Elastomerformkörperausnehmung 32 auf, die in einem Abschnitt 34 des Elastomerformkörpers 3 angeordnet ist, der durch elastische Verbindungselemente 36 in x-y-Richtung gegenüber dem restlichen Elastomerformkörper 3 beweglich angeordnet ist.

Weiterhin ist die Elastomerformkörperausnehmung 32 als Langloch ausgebildet um eine zusätzliche Bewegung eines Abschnitts einer Verbindungseinrichtung 4 zu ermöglichen.

Weiterhin dargestellt ist eine Nut 35 zur Führung der Drehbewegung, genauer zur Führung der Verschiebung der Drehachse. In dieser Nut 35 dargestellt ist ein oben bereits beschriebener Zapfen 5.

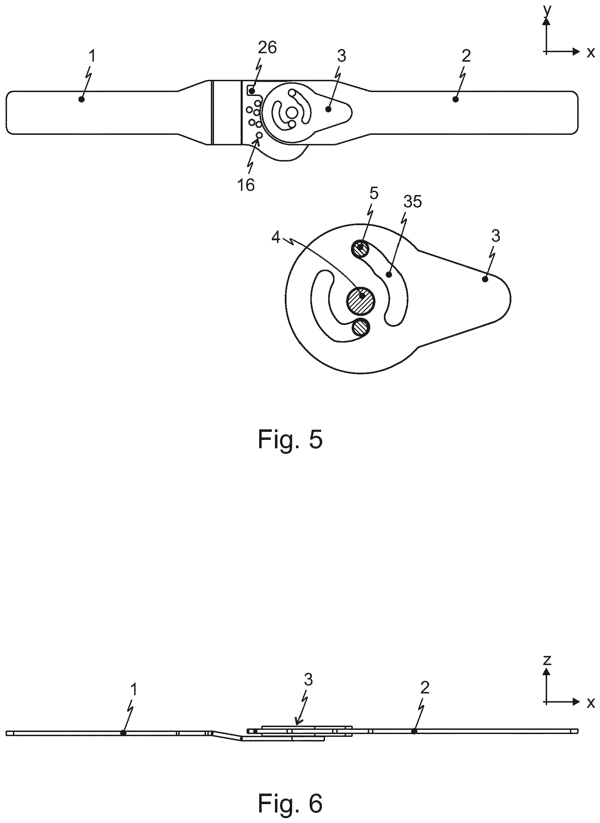

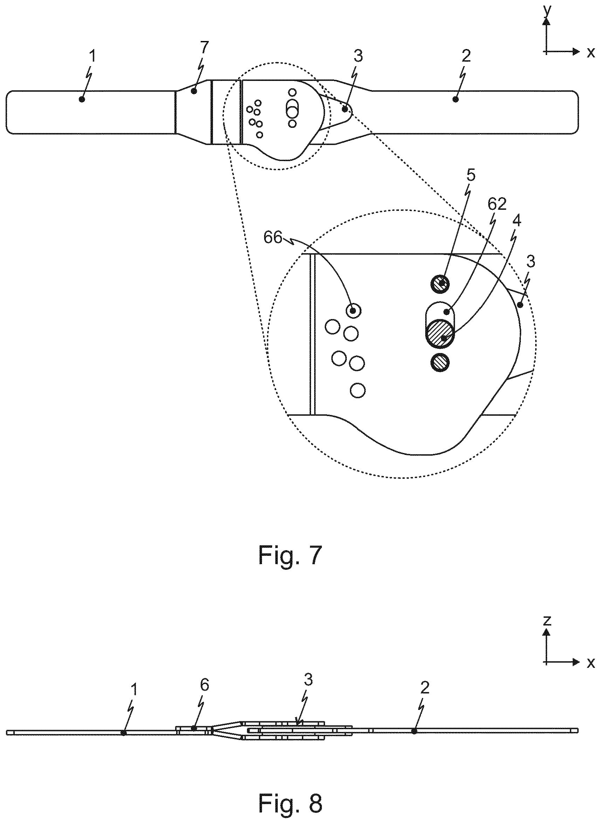

Figuren 5 bis 8 zeigen verschiedene Ansichten einer Gelenkeinrichtung der zweiten erfindungsgemäßen Art mit einem ersten Gelenkarm 1, mit einem zweiten Gelenkarm 2 mit in einer Ausnehmung angeordnetem zweiten Teilkörper eines Elastomerformkörpers 3 und mit einem dritten Teilgelenkarm 7. Figur 5 zeigt hierbei die Draufschicht ohne den dritten Teilgelenkarm. Figur 6 zeigt die zugehörige seitliche Ansicht. Figur 7 zeigt hierbei die Draufschicht mit dem dritten Teilgelenkarm. Figur 8 zeigt die zugehörige seitliche Ansicht.

In Figur 5 dargestellt ist der erste Gelenkarm 1 mit einer Mehrzahl von Arretierausnehmungen 16 in seinem ersten Endabschnitt 10 zur Anordnung von Arretierungsstiften, die eine Drehbewegung bei verschiedenen Drehwinkeln beschränken können. Weiterhin dargestellt ist ein zweiter Gelenkarm 2 mit in der Ausnehmung angeordnetem zweiten Teilkörper des Elastomerformkörpers 3. Dieser ist nochmals vergrößert dargestellt und weist eine Elastomerformkörperausnehmung 32 durch die ein Abschnitt der Verbindungseinrichtung 4, die hier als nicht explizit dargestellte Schraubverbindung ausgebildet ist.

Weiterhin weist der Elastomerformkörper 3 zwei Nuten 35 auf, in denen jeweils ein Zapfen 5 angeordnet ist, wobei die jeweiligen Zapfen 5 vom ersten zu dritten Endabschnitt 10,60 also auch durch die zugeordnete Nut 35 des Elastomerformkörpers 3 hindurchreichen. Im Rahmen der Drehung mit der ersten Normalenrichtung als Drehachse, also im Wesentlichen, oder in anderen Worten taumelnd um die z-Achse, bestimmen diese Zapfen 5, die sich in den zugeordneten Nuten 35 bewegen die Lage der Drehachse relativ zum ersten Gelenkarm 1.

Der zweite Gelenkarm 2 weist als Teil seines zweiten Endabschnitts 20 eine Arretiernase 26 auf, die mit den Arretierungsstiften zusammenwirkt und die Drehbewegung um die z-Achse beschränken.

In Figur 7 und 8 ist jeweils zusätzlich der dritte Teilgelenkarm 70 dargestellt. In Figur 7 samt Ausschnittsvergrößerung sind die Arretierausnehmungen 66 dargestellt, in die ein Arretierungsstift angeordnet werden kann. Ebenfalls dargestellt ist dritte Endabschnittausnehmung 62, die identisch der ersten Endabschnittausnehmung 12 ist und jeweils als Langloch ausgebildet ist. um eine Bewegung der Verbindungseinrichtung 4 innerhalb dieser Endabschnittausnehmungen 12,62 bei Drehbewegung zu erlauben.

## Patentansprüche

1. Gelenkeinrichtung zur Stabilisierung eines bikondylären Gelenks des menschlichen oder tierischen Körpers mit einem ersten und einem zweiten Gelenkarm (1, 2), mit einem Elastomerformkörper (3) und mit einer Verbindungseinrichtung (4),
wobei der erste Gelenkarm (1) einen ersten Endabschnitten (10) mit einer erste Hauptfläche (100), die eine ersten Normalenrichtung (N1) aufweist, und die einen ersten Endabschnittausnehmung (12) aufweist,
wobei der zweite Gelenkarm (2) einen zweiten Endabschnitt (20) mit einer zweiten Hauptfläche (200), die eine zweiten Normalenrichtung (N2) aufweist, die mit der ersten Normalenrichtung (N1) einen Zwischenwinkel (Z) einschließt und die eine zweite Endabschnittausnehmung (22) aufweist, und wobei der der erste und zweite Gelenkarm (1,2) im Bereich ihrer jeweiligen Endabschnitte (10,20) überlappend angeordnet sind,
wobei der Elastomerformkörper (3) einen ersten Teilkörper aufweist, der im Bereich der Endabschnitte (10,20) der Gelenkarme (1,2) zwischen diesen angeordnet ist und eine Elastomerformkörperausnehmung (32) aufweist, die mit der ersten Endabschnittausnehmung (12,22) zumindest teilweise in erster Normalenrichtung (N1) fluchtet
wobei die Verbindungseinrichtung (4) den ersten Gelenkarm (1) zum zweiten Gelenkarm (2) derart beweglich fixiert, dass die Gelenkarme (1,2) gegeneinander um die erste Normalenrichtung (N1) als Drehachse der Bewegung des Gelenks folgend verdrehbar sind und hierzu einer der Gelenkarme (1,2) in seinem Endabschnitt (10.20) einen Zapfen (5) aufweist, der in eine Nut (35) des Elastomerformkörpers (3) eingreift und während der Bewegung dieser Nut (35) folgend die Form der Bewegung bestimmt und dass hierbei die beiden Gelenkarme (1,2) eine Kippbewegung zueinander ausführen können, wobei sich der Wert des Zwischenwinkels (Z) verändert.

2. Gelenkeinrichtung nach Anspruch 1, wobei
die Verbindungseinrichtung (4) einen Abschnitt aufweist, der durch die erste Endabschnittausnehmung (12) und die Elastomerformkörperausnehmung (32) hindurchreicht.

3. Gelenkeinrichtung nach Anspruch 2, wobei
die Elastomerformkörperausnehmung (32) senkrecht zur ersten Normalenrichtung (N1) beweglich in dem Elastomerformkörper (3) angeordnet ist.

4. Gelenkeinrichtung nach Anspruch 2 oder 3, wobei
die erste Endabschnittausnehmung (12) eine Ausdehnung senkrecht zur ersten Normalenrichtung (N1) aufweist, die größer ist als der darin angeordnete Abschnitt der Verbindungseinrichtung (4).

5. Gelenkeinrichtung nach einem der vorhergehenden Ansprüche, wobei der Elastomerformkörper (3) ausgebildet ist entweder aus einem Material der Materialgruppe der Elastomere, bevorzugt der Silikonkautschuke, insbesondere aus thermisch stabilisiertem Silikonkautschuk, insbesondere mit einer Shore-A-Härte zwischen 55 und 90, bevorzugst zwischen 70 und 80 oder aus einem Material der Materialgruppe der modifizierten PTFEs, bevorzugt der gefülltem PTFE, insbesondere aus mit Silikat gefülltem PTFE, insbesondere mit einem Kaltstauchwert zwischen 3% und 40%, insbesondere zwischen 3% und 20%.

6. Gelenkeinrichtung nach einem der vorhergehenden Ansprüche, wobei ein zweiter Teilkörper des Elastomerformkörpers (3) in einer Ausnehmung (23) eines Endabschnitts (20) eines Gelenkarms (2), vorzugsweise verdrehsicher, angeordnet ist.

7. Gelenkeinrichtung nach einem der vorhergehenden Ansprüche, wobei ein dritter Gelenkarm (6) oder Teilgelenkarm (7) mit einem dritten Endabschnitt (60) an dem ersten Gelenkarm (1) fixiert angeordnet ist, wobei zweiten Endabschnitt (20) und der Elastomerformkörper (3) zwischen dem ersten und dritten Endabschnitt (10,20) angeordnet sind.

8. Gelenkeinrichtung nach einem der vorhergehenden Ansprüche, wobei die Verbindungseinrichtung (4) als Schraubverbindung oder als Nietverbindung ausgebildet ist.

9. Orthese mit einer ein bikondyläres Gelenk nachbildenden Gelenkeinrichtung gemäß einem der vorhergehenden Ansprüche und mit einer ersten und einer zweiten Manschette, die jeweils eine Fixiereinrichtung zur Fixierung der Orthese an einer Extremität mit einem bikondylären Gelenk, das eine Gelenkdrehachse aufweist, des menschlichen oder tierischen Körpers aufweisen, wobei die erste Manschette mittels der Gelenkeinrichtung mit der zweiten Manschette verbunden ist und die erste und zweite Normalenrichtung im Wesentlichen parallel zur Gelenkdrehachse ausgerichtet sind..

10. Orthese nach Anspruch 9, wobei
gegenüberliegend jeweils eine Gelenkeinrichtung angeordnet ist.

11. Orthese nach Anspruch 9 oder 10, wobei
die Orthese als Knieorthese, die erste Manschette als Oberschenkelmanschette und die zweite Manschette als Unterschenkelmanschette ausgebildet sind.

## Claims

1. Joint device for stabilising a bicondylar joint of the human or animal body with a first and a second articulated arm (1, 2), with a moulded elastomer body (3) and with a connecting device (4), whereby the first articulated arm (1) has a first end section (10) with a first main area (100), which has a first normal direction (N1), and which has a first end section recess (12), whereby the second articulated arm (2) has a second end section (20) with a second main area (200), which has a second normal direction (N2) which, with the first normal direction (N1), encloses an intermediate angle (Z) and which has a second end section recess (22), and whereby the first and second articulated arm (1,2) are arranged to overlap in the area of their respective end sections (10,20), whereby the moulded elastomer body (3) has a first partial body which, in the area of the end sections (10,20) of the articulated arms (1,2), is arranged between them and has a moulded elastomer body recess (32), which is at least partly aligned with the first end section recess (12,22) in the first normal direction (N1) whereby the connecting device (4) movably fixes the first articulated arm (1) to the second articulated arm (2) in such a way that the articulated arms (1,2) are rotatable in opposite directions about the first normal direction (N1) as the axis of rotation following the movement of the joint and to this end one of the articulated arms (1,2) has a pin (5) in its end section (10.20), which engages in a groove (35) of the moulded elastomer body (3) and during the movement following this groove (35) determines the form of the movement and in doing so the two articulated arms (1,2) can perform a tilting movement to each other, whereby the value of the intermediate angle (Z) changes.

2. Joint device according to Claim 1, whereby the connecting device (4) has a section, which passes through the first end section recess (12) and the moulded elastomer body recess (32).

3. Joint device according to Claim 2, whereby the moulded elastomer body recess (32) is arranged perpendicular to the first normal direction (N1) movable in the moulded elastomer body (3).

4. Joint device according to Claim 2 or 3, whereby the first end section recess (12) has an extension perpendicular to the first normal direction (N1), which is larger than the second of the connecting device (4) arranged in it.

5. Joint device according to one of the preceding claims, whereby the moulded elastomer body (3) is formed either from a material of the elastomers material group, preferably silicone rubbers, in particular from thermally stabilised silicone rubber, in particular with a Shore A hardness between 55 and 90, preferably between 70 and 80 or made from a material of the modified PTFEs material group, preferably the filled PTFE, in particular from PTFE filled with silicate, in particular with a cold compression value between 3% and 40%, in particular between 3% and 20%.

6. Joint device according to one of the preceding claims, whereby a second partial body of the moulded elastomer body (3) is arranged in a recess (23) of an end section (20) of an articulated arm (2), preferably torsionally resistant.

7. Joint device according to one of the preceding claims, whereby a third articulated arm (6) or partial articulated arm (7) with a third end section (60) is arranged fixed on the first articulated arm (1), whereby the second end section (20) and the moulded elastomer body (3) are arranged between the first and third end section (10,20).

8. Joint device according to one of the preceding claims, whereby the connecting device (4) is formed as a threaded connection or as a rivet joint.

9. Orthosis with a joint device replicating a bicondylar joint according to one of the preceding claims and with a first and a second cuff, which each have a fixing device for fixing the orthosis on an extremity with a bicondylar joint, which has a joint axis of rotation, of the human or animal body, whereby the first cuff is connected to the second cuff by means of the joint device and the first and second normal direction are essentially aligned parallel with the joint axis of rotation.

10. Orthosis according to Claim 9, whereby one joint device each is arranged opposite.

11. Orthosis according to Claim 9 or 10, whereby the orthosis is formed as a knee orthosis, the first cuff as a thigh cuff and the second cuff as a lower leg cuff.

## Revendications

1. Dispositif d'articulation permettant de stabiliser une articulation bicondylienne chez l'homme ou chez l'animal avec un premier et un deuxième bras d'articulation (1, 2), avec un moulage en élastomère (3) et avec un dispositif de liaison (4), alors que le premier bras d'articulation (1) possède une première section d'extrémité (10) avec une première surface principale (100), qui a une première direction normale (N1), et qui a un évidement au niveau de la première section d'extrémité (12), alors que le deuxième bras articulé (2) possède une deuxième section d'extrémité (20) avec une deuxième surface principale (200), qui a une deuxième direction normale (N2), qui forme un angle intermédiaire (Z) avec la première direction normale (N1), et qui présente un deuxième évidement au niveau de la section d'extrémité (22), alors que les premier et deuxième bras articulés (1,2) sont disposés en chevauchement au niveau de leurs sections d'extrémité respectives (10,20), alors que le moulage en élastomère (3) a un premier moulage partiel qui est positionné dans la région des sections d'extrémité (10,20) des bras articulés (1,2) entre eux et a un évidement au niveau du moulage en élastomère (32) qui est au moins partiellement aligné avec le premier évidement de la section d'extrémité (12,22) dans la première direction normale (N1), le dispositif de liaison (4) fixant de manière mobile le premier bras articulé (1) au Deuxième bras articulé (2) de telle sorte que les bras articulés (1,2) puissent être tournés l'un par rapport à l'autre autour de la première direction normale (N1) qui fait office d'axe de rotation du mouvement de l'articulation, et, à cet effet, l'un des bras articulés (1,2) comporte un tenon (5) au niveau de sa section d'extrémité (10.20) lequel s'engage dans une rainure (35) du moulage en élastomère (3) et détermine la forme du mouvement pendant le mouvement de cette rainure (35), ce qui fait qu'ainsi, les deux bras articulés (1,2) peuvent basculer l'un vers l'autre, alors que la valeur de l'angle intermédiaire (Z) change.

2. Dispositif d'articulation selon la revendication 1, alors que le dispositif de liaison (4) possède un évidement qui traverse le premier évidement au niveau de la section d'extrémité (12) et l'évidement au niveau du moulage en élastomère (32).

3. Dispositif d'articulation se référant à la revendication 2, alors que l'évidement au niveau du moulage en élastomère (32) est positionné verticalement par rapport à la première direction normale (N1) et de manière mobile dans le moulage en élastomère (3).

4. Dispositif d'articulation selon la revendication 2 ou 3, alors que le premier évidement au niveau de la section d'extrémité (12) possède un évidement verticalement par rapport à la première direction normale (N1) qui est plus grand que la section du dispositif de liaison (4) située à l'intérieur.

5. Dispositif d'articulation selon l'une des revendications précédentes, alors que le moulage en élastomère (3) est constitué soit d'un matériau appartenant à la catégorie des élastomères, de préférence en caoutchouc de silicone, et notamment en caoutchouc de silicone stabilisé thermiquement, et notamment avec un degré de dureté Shore-A entre 55 et 90, et de préférence entre 70 et 80, soit d'un matériau appartenant à la catégorie des PTFE modifiés, et de préférence en PTFE rempli et notamment en PTFE rempli de silicate, et notamment avec une valeur de frappe à froid entre 3 % et 40 %, et notamment entre 3 % et 20 %.

6. Dispositif d'articulation selon l'une des revendications précédentes, alors qu'un deuxième moulage partiel du moulage en élastomère (3) est positionné dans un évidement (23) d'une section d'extrémité (20) d'un bras d'articulation (2), et de préférence sans pouvoir pivoter.

7. Dispositif d'articulation selon l'une des revendications précédentes, alors qu'un troisième bras d'articulation (6) ou bras d'articulation partiel (7) est positionné de manière fixe avec une troisième section d'extrémité (60) au niveau du premier bras d'articulation (1), alors que la deuxième section d'extrémité (20) et le moulage en élastomère (3) sont positionnés entre la première et la troisième section d'extrémité (10,20).

8. Dispositif d'articulation selon l'une des revendications précédentes, alors que le dispositif de liaison (4) constitue une liaison vissée ou une liaison rivetée.

9. Orthèse avec dispositif d'articulation imitant une articulation bicondylienne selon l'une des revendications précédentes et muni d'un premier et d'un second manchon formant chacun un dispositif de fixation permettant de fixer l'orthèse au niveau d'une extrémité présentant une articulation bicondylienne qui possède un axe d'articulation, chez l'homme ou chez l'animal, alors que le premier manchon est relié au second manchon à l'aide d'un dispositif d'articulation et que la première et la seconde direction normale sont à la base positionnées en parallèle par rapport à l'axe de pivotement de l'articulation.

10. Orthèse selon la revendication 9, alors qu'un dispositif d'articulation a été placé en face.

11. Orthèse selon la revendication 9 ou 10, alors que l'orthèse est une orthèse pour le genou, le premier manchon faisant office de manchon pour la cuisse et le deuxième manchon faisant office de manchon pour la jambe.
